# EUROPEAN PATENT SPECIFICATION

(11) **EP 3 679 037 B1**
(45) Date of publication and mention of the grant of the patent: **08.11.2023**
(21) Application number: 18849896.8
(22) Date of filing: 04.09.2018
(51) Int. Cl.: C07D 417/12, C07D 407/12, C07D 403/12, C07D 257/06, A61K 31/41, A61K 31/427, A61K 31/4196, A61P 31/04, A61P 31/10, C07D 405/12

(54) **NEW 5-AMINOTETRAZOLE DERIVATIVES WITH ANTIBACTERIAL AND ANTIFUNGAL ACTIVITY**
NEUE 5-AMINOTETRAZOL-DERIVATE MIT ANTIBAKTERIELLER UND ANTIMYKOTISCHER AKTIVITÄT
NOUVEAUX DÉRIVÉS DE 5-AMINOTÉTRAZOLE AYANT UNE ACTIVITÉ ANTIBACTÉRIENNE ET ANTIFONGIQUE

(30) Priority: 04.09.2017 PL 42274117
(43) Date of publication of application: 15.07.2020
(73) Proprietor: Warszawski Uniwersytet Medyczny, 02-091 Warszawa (PL)
(72) Inventor: SZULCZYK, Daniel, 05-250 Slupno (PL); STRUGA, Marta, 05-250 Slupno (PL); STEFANSKA, Joanna, 05-250 Slupno (PL)
(74) Representative: Witek, Rafal
(86) International application number: PCT/PL2018/050047
(87) International publication number: WO 2019/045581

(56) References cited:
- EP-A1- 0 855 394
- EP-A1- 0 855 394
- CN-A- 105 503 762
- US-A1- 2012 329 772
- SZULCZYK DANIEL ET AL: "Design and synthesis of novel 1H-tetrazol-5-amine based potent antimicrobial agents: DNA topoisomerase IV and gyrase affinity evaluation supported by molecular docking studies", EUROPEAN JOURNAL OF MEDICINAL CHEMISTRY, vol. 156, 1 August 2018 (2018-08-01), pages 631-640, XP055799107, AMSTERDAM, NL ISSN: 0223-5234, DOI: 10.1016/j.ejmech.2018.07.041
- BIELENICA ANNA ET AL: "1H-Tetrazol-5-amine and 1,3-thiazolidin-4-one derivatives containing 3-(trifluoromethyl)phenyl scaffold: Synthesis, cytotoxic and anti-HIV studies", BIOMEDICINE AND PHARMACOTHERAPY., vol. 94, 1 October 2017 (2017-10-01), pages 804-812, XP055799179, FR ISSN: 0753-3322, DOI: 10.1016/j.biopha.2017.07.152
- X RAMESH YELLA et al.: "Tandem regioselective synthesis of tetrazoles A and related heterocycles using iodine", Organic & Biomolecular Chemistry, vol. 9, 2011, pages 3235-3245, XP055578638,
- X SRIMANTA GUIN et al.: "Desulfurization Strategy in the Construction of A Azoles Possessing Additional Nitrogen, Oxygen or Sulfur using a Copper(l) Catalyst", Advanced Synthesis & Catalysis, vol. 354, 2012, pages 2757-2770, XP055578647,
- X YUANYUAN XIE et al.: "An efficient method for the synthesis of A substituted 5-aminotetrazoles from selenoureas using Phl(OAc)2", Tetrahedron Letters, vol. 56, 2015, pages 2533-2536, XP055579947,

## Description

The present invention relates to novel 5-aminotetrazole derivatives with antibacterial and antifungal activity.

Bielenica Anna et al. in publication "1H-Tetrazol-5-amine and 1,3-thiazolidin-4-one derivatives containing 3-(trifluoromethyl)phenyl scaffold: Synthesis, cytotoxic and anti-HIV studies" Biomedicine and Pharmacotherapy, vol. 94, October 2017, pages 804-812, disclose compounds including phenyl rings and their cytotoxic and anti-HIV activity.

EP 855 394 A1 describes similar compounds including phenyl rings, which are disclosed as microbiocides, insecticides and herbicides.

Despite the constant development of pharmacy, there is still a need for new antibiotics or antifungal compounds. It results from the progressive drug resistance of microorganisms to used antibiotics and antifungal drugs. The present invention relates to a compound of the formula I 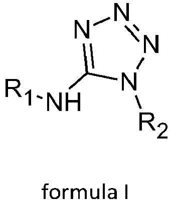 wherein R₁ and R₂ are independently aromatic, heteroaromatic ring, or aliphatic, nonaromatic substituents, especially phenyl, triazole, thiadiazole or furan, substituted with halogen, especially selected from F, Cl, Br, or methyl, trifluoromethyl, methoxy or ethoxy group, wherein said compound has been selected from:

N-(1-(3,4-dichlorophenyl)-1H-tetrazol-5-yl)thiazole-2-amine (1),

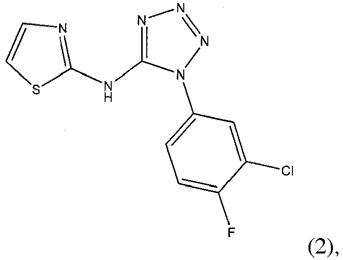

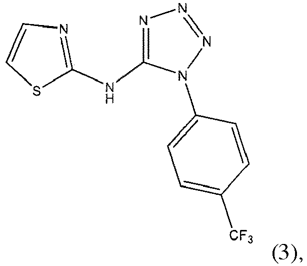

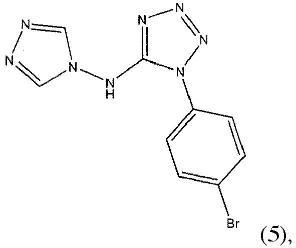

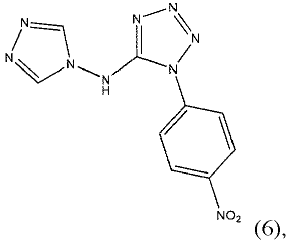

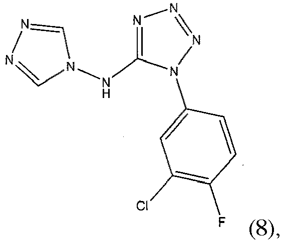

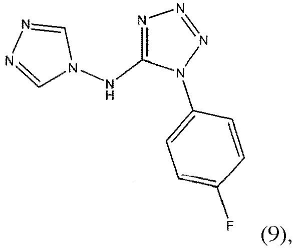

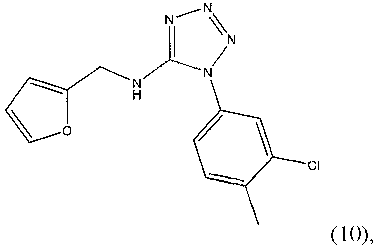

or

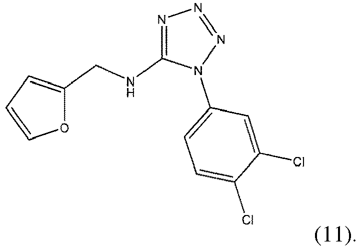

Another object of the invention is the compound of the invention as defined above for use as a medicament, preferably, as an antibacterial drug, preferably, as an antifungal drug.

5-Aminotetrazoles with the above-mentioned activity are described by the general formula, where R₁ and R₂ are substituted by halogen (F, Cl, Br) or another group (methyl, trifluoromethyl, methoxy, ethoxy) aromatic/non-aromatic rings (phenyl/cyclohexyl), aliphatic or heteroaromatic substituents (triazole, thiadiazole, furan).

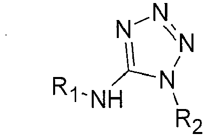

These compounds were obtained from 1,3-disubstituted thiourea by reaction with sodium azide in the presence of mercuric chloride and triethylamine. Dimethylformamide was used as a solvent. The compounds are obtained at room temperature, by mixing the reagents and purification on a chromatography column.

### Examples 1-11. Preparation of compound 1-11.

### Compound 1.

### N-(1-(3,4-dichlorophenyl)-1H-tetrazol-5-yl)thiazole-2-amine, C₁₀H₆Cl₂N₆S, M= 313,16 g/mol

Sodium azide (NaN₃ - 3.75 mmol), mercuric chloride (HgCh - 1.38 mmol) and triethylamine (5 ml) were added to a solution of 1-(3,4-dichlorophenyl)-3-(thiazol-2-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 1.

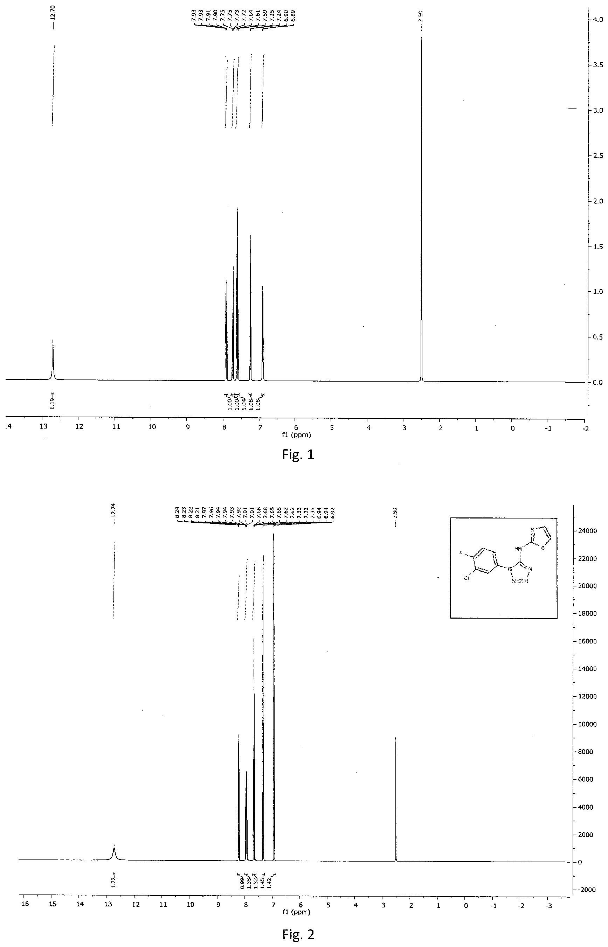

The structure was confirmed by ¹H NMR (Fig. 1).

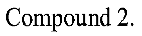

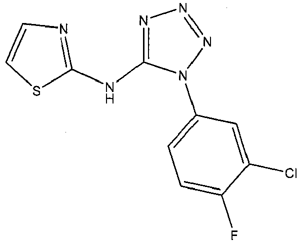

### N-(1-(3-chloro-4-fluorophenyl)-1H-tetrazol-5-yl)thiazol-2-amine, C₁₀H₆ClFN₆S, M= 296,71 glmol

Sodium azide (NaN₃ - 3.75 mmol), mercuric chloride (HgCh - 1.38 mmol) and triethylamine (5 ml) were added to a solution of 1-(3-chloro-4-fluorophenyl)-3-(thiazol-2-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 2.

The structure was confirmed by ¹H NMR (Fig. 2).

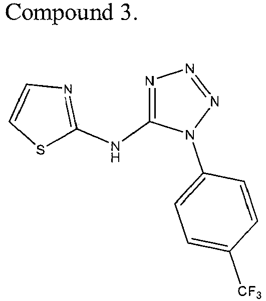

### N-(1-(4-(trifluoromethyl)phenyl)-1H-tetrazol-5-yl)thiazole-2-amine, C₁₁H₇F₃N₆S, 312,27 g/mol

Sodium azide (NaN₃ - 3.75 mmol), mercuric chloride (HgCh - 1.38 mmol) and triethylamine (5 ml) were added to a solution of 1-(thiazol-2-yl)-3-(4-(trifluoromethyl)phenyl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 3.

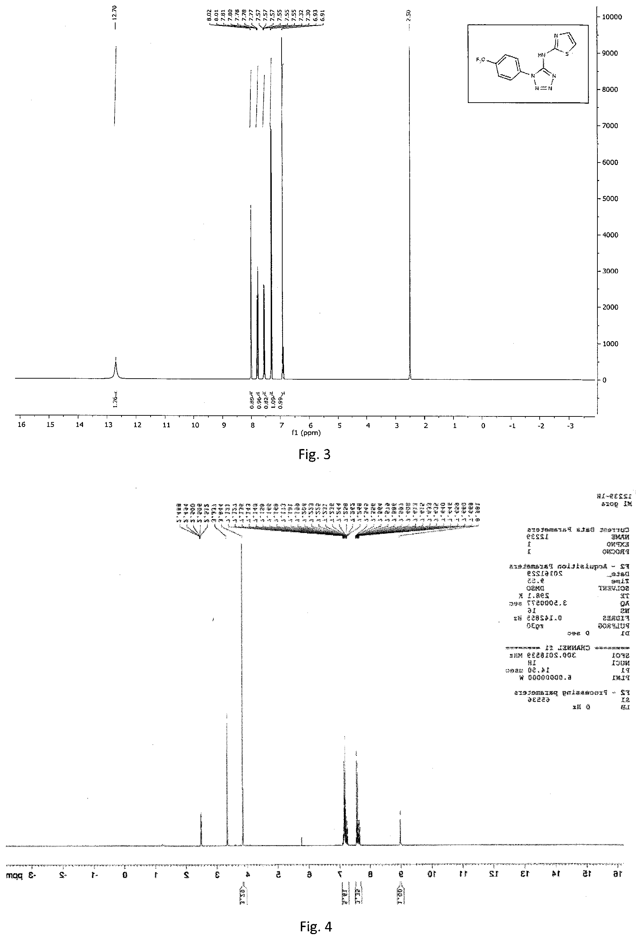

The structure was confirmed by ¹H NMR (Fig. 3).

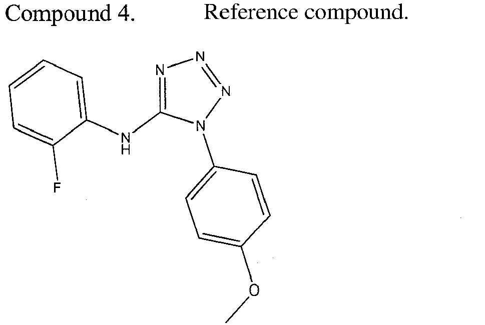

### (N-(2-fluorophenyl)-1-(4-methoxyphenyl)-1H-tetrazol-5-amine), C₁₄H₁₂FN₅O, M= 285.28 g/mol

Sodium azide (NaN₃ - 3.75 mmol), mercuric chloride (HgCl₂ - 1.38 mmol) and triethylamine (5 ml) were added to a solution of 1-(2-fluorophenyl)-3-(4-methoxyphenyl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 4. The structure was confirmed by ¹H NMR (Fig. 4).

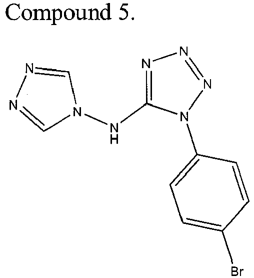

### 1-(4-bromophenyl)-N-(4H-1,2.4-triazol-4-yl)-1H-tetrazole-5-amine, C₉H₇BrN₈, M= 307.12 g/mol

Sodium azide (NaN₃ - 3.75 mmol), mercuric chloride (HgCh - 1.38 mmol) and triethylamine (5 ml) were added to a solution of 1-(4-bromophenyl)-3-(4H-1,2,4-triazol-4-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 5.

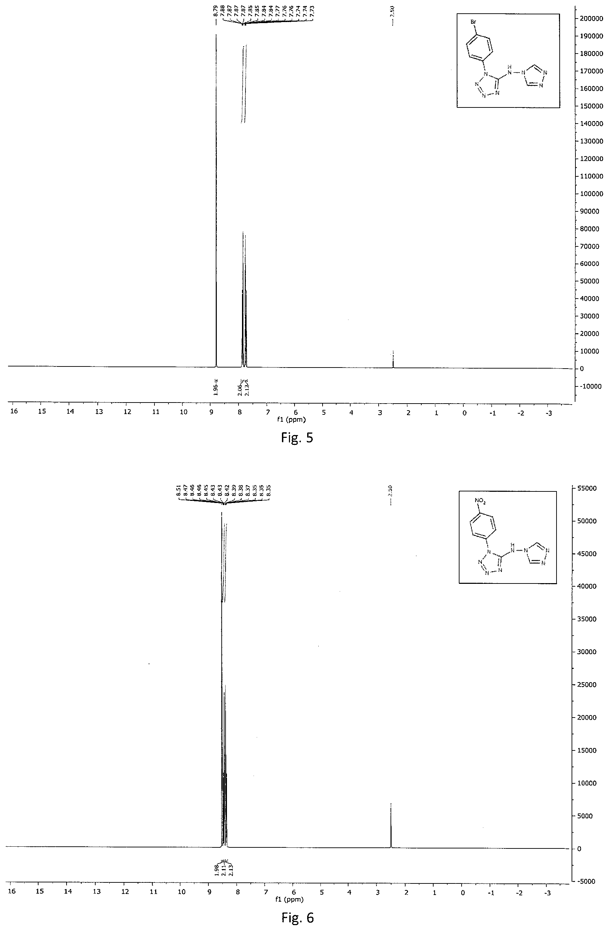

The structure was confirmed by ¹H NMR (Fig. 5).

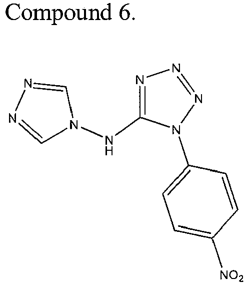

### 1-(4-nitrophenyl)-N-(4H-1,2.4-triazol-4-yl)-1H-tetrazol-5-amine, C₉H₇N₉O₂, M= 273.22 g/mol

Sodium azide (NaN₃ - 3.75 mmol), mercuric chloride (HgCl₂ - 1.38 mmol) and triethylamine (5 ml) were added to a solution of 1-(4-nitrophenyl)-3-(4*H*-1,2,4-triazol-4-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 6.

The structure was confirmed by ¹H NMR (Fig. 6).

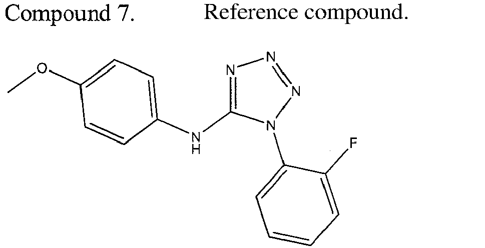

### 1-(2-fluorophenyl)-N-(4-methoxyphenyl)-1H-tetrazol-5-amine), C₁₄H₁₂FN₅O, M= 285.28 g/mol

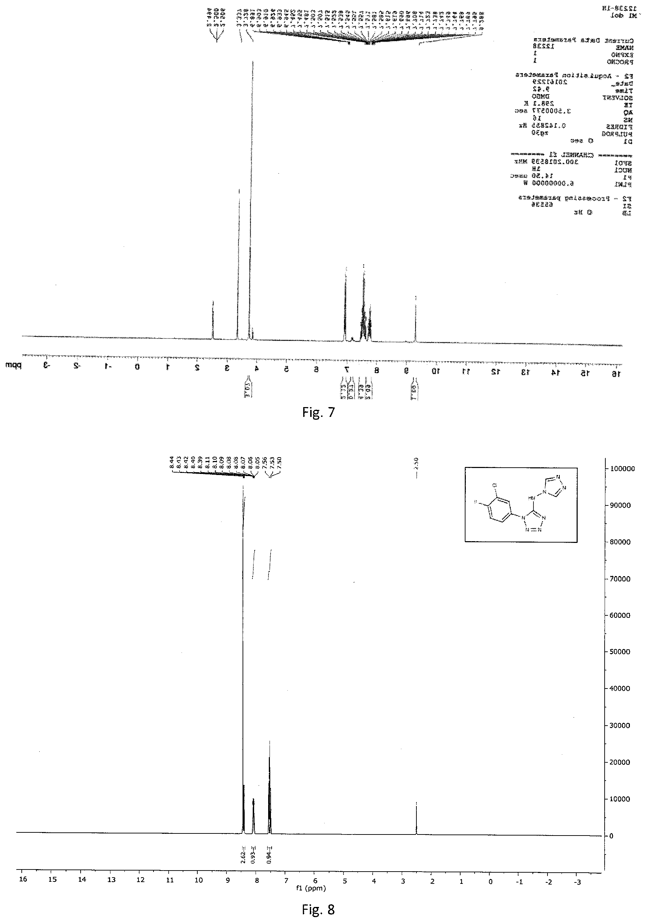

Sodium azide (NaN₃ - 3.75 mmol), mercuric chloride (HgCh - 1.38 mmol) and triethylamine (5 ml) were added to a solution of 1-(2-fluorophenyl)-3-(4-methoxyphenyl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 7. The structure was confirmed by ¹H NMR (Fig. 7).

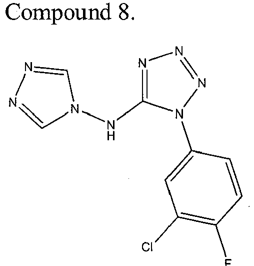

### 1-(3-chloro-4-fluorophenyl)-N-(4H-1,2.4-triazol-4-yl)-1H-tetrazole-5-amine, C₉H₆ClFN₈, M= 280.65 g/mol

Sodium azide (NaN₃ - 3.75 mmol), mercuric chloride (HgCh - 1.38 mmol) and triethylamine (5 ml) were added to a solution of 1-(3-chloro-4-fluorophenyl)-3-(4*H*-1,2,4-triazol-4-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 8.

The structure was confirmed by ¹H NMR (Fig. 8).

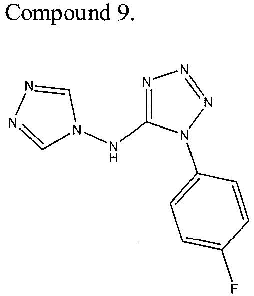

### 1-(4-fluorophenyl)-N-(4H-1,2.4-triazol-4-yl)-1H-tetrazole-5-amine, C₉H₇FN₈, M= 246.21 g/mol

Sodium azide (NaN₃ - 3.75 mmol), mercuric chloride (HgCl₂ - 1.38 mmol) and triethylamine (5 ml) were added to a solution of 1-(4-fluorophenyl)-3-(4*H*-1,2,4-triazol-4-yl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 9.

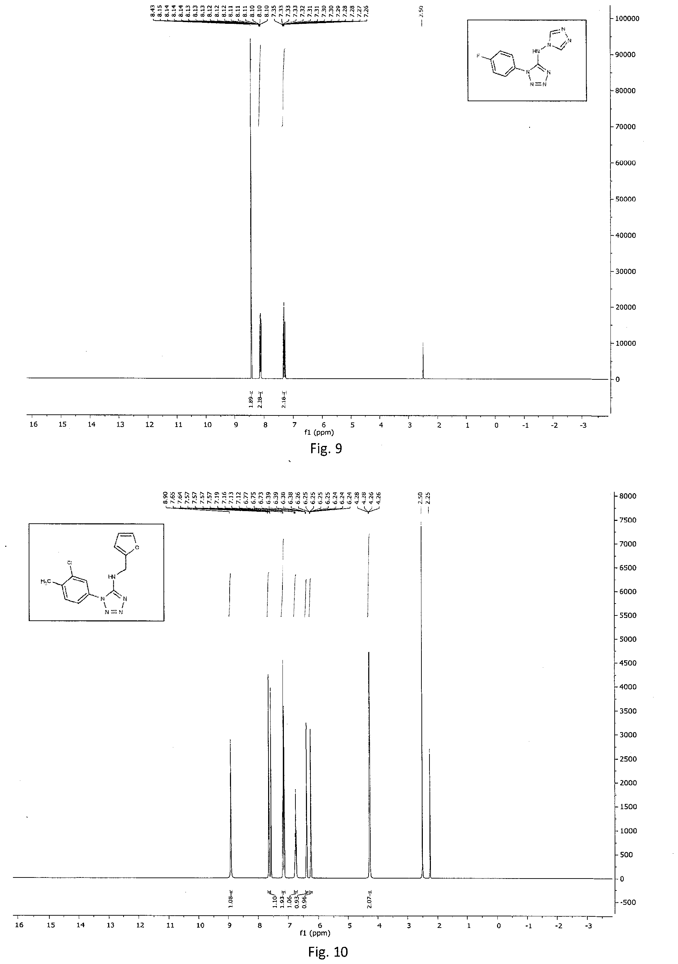

The structure was confirmed by ¹H NMR (Fig. 9).

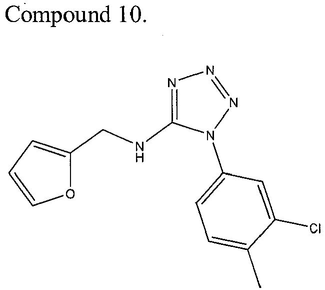

### 1-(3-chloro-4-methylphenyl)-N-(furan-2-ylmethyl)-1H-tetrazole-5-amine, C₁₃H₁₂ClN₅O, M= 289.72 g/mol

Sodium azide (NaN₃ - 3.75 mmol), mercuric chloride (HgCh - 1.38 mmol) and triethylamine (5 ml) were added to a solution of 1-(3-chloro-4-methylphenyl)-3-(furan-2-ylmethyl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 10.

The structure was confirmed by ¹H NMR (Fig. 10).

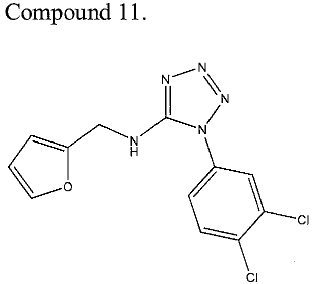

### 1-(3,4-dichlorophenyl)-N-(furan-2-ylmethyl)-1H-tetrazole-5-amine, C₁₂H₉Cl₂N₅O, M= 310.14 g/mol

Sodium azide (NaN₃ - 3.75 mmol), mercuric chloride (HgCh - 1.38 mmol) and triethylamine (5 ml) were added to a solution of 1-(3,4-dichlorophenyl)-3-(furan-2-ylmethyl)thiourea (1 mmol) in dimethylformamide (DMF - 5 ml). The prepared solution was stirred for 6 hours on a magnetic stirrer at room temperature. The mixture was then filtered through a paper filter. The precipitate on the filter was rinsed with 3 portions (5 ml) of chloroform. The resulting solution was evaporated on an evaporator. The obtained precipitate was purified by means of column chromatography (chloroform:methanol 9.5:0.5 vol.), thereby obtaining purified compound 11.

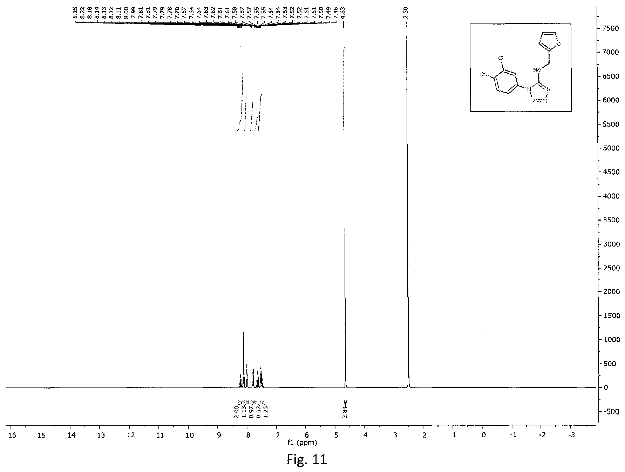

The structure was confirmed by ¹H NMR (Fig. 11).

**The list of structures of compounds 1 - 11.**

| 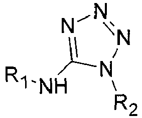 | | | | | |
|---|---|---|---|---|---|
| **Compound** | **R₁** | **R₂** | **Compound** | **R₁** | **R₂** |
| 1 | 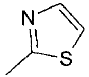 | 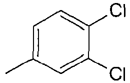 | 7_{(∗)} | 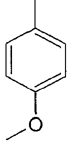 | 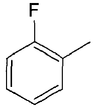 |
| 2 | 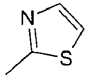 | 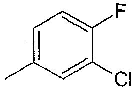 | 8 | 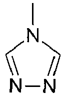 | 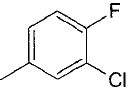 |
| 3 | 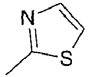 | 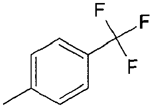 | 9 | 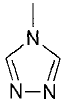 | 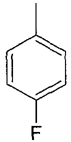 |
| 4 (*) | 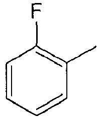 | 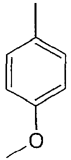 | 10 | 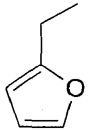 | 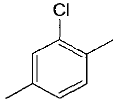 |
| 5 | 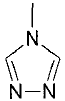 | 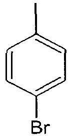 | 11 | 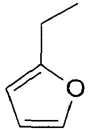 | 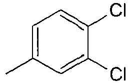 |
| 6 | 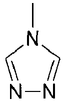 | 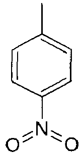 | | | |

| | | | | | |
|---|---|---|---|---|---|
| (*) Reference compounds | | | | | |

### Example 12. Biological activity of the compounds according to the invention.

The study of the antimicrobial activity of the derivatives was carried out using standard bacterial strains and yeast-like fungi, from international microbial collections: ATCC (*American Type Culture Collection* - Rockville, USA) and NCTC (*National Collection of Type Culture* - London, Great Britain). The group of reference strains were Gram-positive (*Staphylococcus aureus* NCTC 4163, *S. aureus* ATCC 25923, *S. aureus* ATCC 6538, *S*. *aureus* ATCC 29213, *S*. *epidermidis* ATCC 12228, *S. epidermidis* ATCC 35984, *Bacillus subtilis* ATCC 6633, *B. cereus* ATCC 11778, *Enterococcus hirae* ATCC 10541, *E. faecalis* ATCC 29212, *Micrococcus luteus* ATCC 10240, *M. luteus* ATCC9341), Gram-negative bacteria (*Escherichia coli* NCTC 10538, *E. coli* ATCC 25922, *P. vulgaris* NCTC 4635, *Pseudomonas aeruginosa* ATCC 15442, *P. aeruginosa* ATCC 27853, *Bordetella bronchiseptica* ATCC 4617) and yeast-like fungi of the genus *Candida* (*C. albicans* ATCC 10231, *C*. *albicans* ATCC 90028, *C. parapsilosis* 22019). These strains are commonly used to determine the activity of chemotherapeutics, disinfectants and to check the sensitivity of bacteria, and their use allows to compare the results obtained in independent laboratories around the world. Clinical isolates of Gram-positive cocci from *Staphylococcus* genus (*S*. *aureus* and *S*. *epidermidis*) and Gram-negative bacilli from *Escherichia coli* and *Pseudomonas aeruginosa* species were also used in the study.

Determination of the smallest concentration of antimicrobial growth inhibitory compound (MIC - Minimal Inhibitors Concentration) was carried out by serial dilution of the test compounds in liquid media as required by the CLSI (*Clinical and Laboratory Standards Institute).*

The bacterial test was carried out in MHII liquid medium using 96-well polystyrene microplates, incubation was carried out for 18 hours at 35 °C. The final bacterial inoculum was about 5×10⁶ CFU/ml (*Colony Forming Unit*), and the compounds were tested in the concentration range of 256-1 µg/ml. The MIC value was the lowest concentration of the test compound at which no bacterial growth was observed.

The performed tests showed a clear antibacterial activity of several derivatives and the obtained MIC values of the most active compounds against Gram-positive bacteria strains were within 1-4 µg/ml. The test compounds also acted as inhibitors for Gram-negative bacilli (MIC 4-8 µg/ml) and fungi of the genus Candida. This activity is comparable to the leading chemotherapeutics used in medicine.

The obtained compounds were tested for potential cytotoxicity. They are not cytotoxic to the non-cancerous, immortalized human keratinocyte (HaCaT) cell line and the lung cancer cell line (A549). The results of cytotoxicity are for all tested compounds above 60 µM (IC₅₀ - value of test concentration at which cell proliferation is inhibited by 50% in relation to the control system).

| | IC₅₀ ( µM) | |
|---|---|---|
| Compound | HaCaT | A549 |
| **2** | 60 | 60 |
| **3** | 80 | 76 |
| **5** | 65 | 62 |
| **6** | 65 | 60 |
| **8** | 62 | 60 |
| **9** | 84 | 80 |
| **10** | 80 | 78 |
| **11** | 84 | 80 |

The mechanism of action was verified for the obtained compounds by inhibiting bacterial topoisomerases (Topoisomerase IV and Gyrase). Obtained derivatives exhibit high efficacy in relation to Gram-positive bacteria, and the research results show that the target of their action are bacterial topoisomerases.

Based on the obtained results, the dose of the inhibitor suppressing 50% activity of topoisomerase IV and gyrase (*Staphylococcus aureus*) was calculated - IC₅₀.

| Compound | IC₅₀ (µg/mL) | |
|---|---|---|
| | *S. aureus* DNA Gyrase | *S*. *aureus* Topoisomerase IV |
| **10** | 22.8 ± 0.4 | 11.9 ± 1.3 |
| **11** | 0.9 ± 0.1 | 2.6 ± 0.2 |
| Ciprofloxacin | 3.5 ± 0.3 | 1.70 ± 0.15 |

The obtained compounds should find application in the production of new antibacterial and antifungal chemotherapeutics with a broad spectrum of activity.

## Claims

1. A compound of formula I 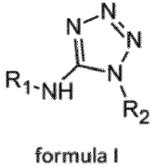 wherein R₁ and R₂ are independently an aromatic ring, in particular a phenyl or heteroaromatic ring, in particular a triazole, thiadiazole or furan, substituted with a halogen, especially one selected from F, Cl, Br, or methyl, trifluoromethyl, methoxy or ethoxy group, wherein said compound has been selected from:
N-(1-(3,4-dichlorophenyl)-1H-tetrazol-5-yl)thiazole-2-amine (1), 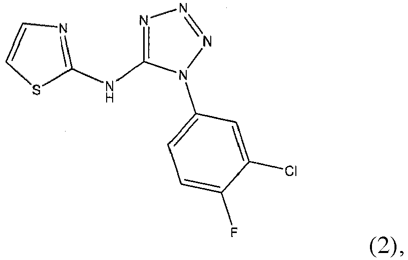 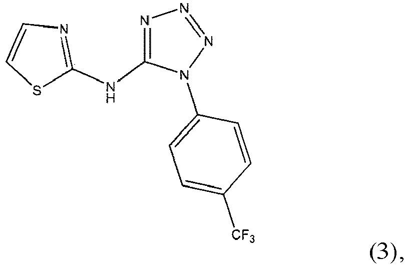 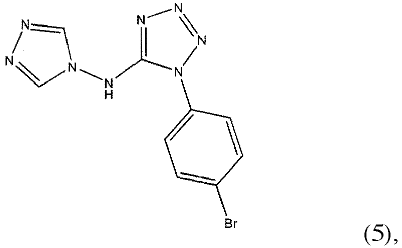 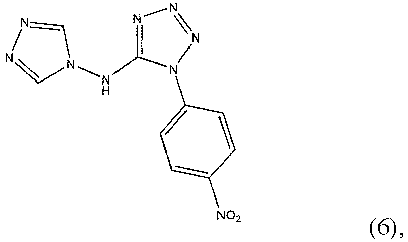 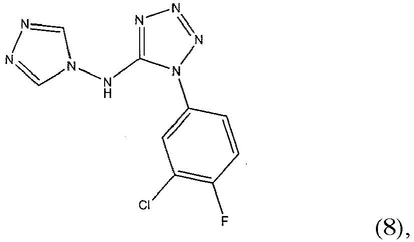 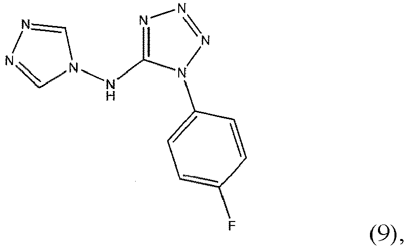 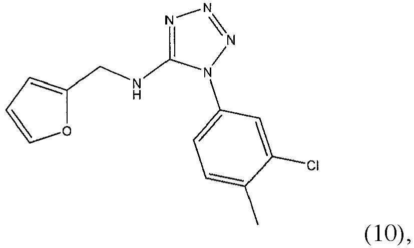
or

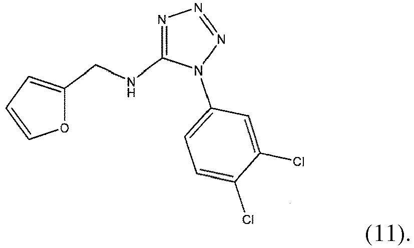

2. The compound according to claim 1 for use as a medicament.

3. The compound according to claim 2 for use as an antibacterial drug.

4. The compound according to claim 2 for use as an antifungal drug.

## Patentansprüche

1. Verbindung der Formel I 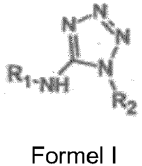 wobei R₁ und R₂ unabhängig ein aromatischer Ring sind, insbesondere ein Phenyl- oder heteroaromatischer Ring, insbesondere ein Triazol, Thiadiazol oder Furan, das mit einem Halogen, insbesondere einem, das aus F, Cl, Br ausgewählt ist, oder einer Methyl-, Trifluormethyl-, Methoxy- oder Ethoxygruppe substituiert ist, wobei die Verbindung ausgewählt ist aus:
N-(1-(3,4-dichlorphenyl)-1H-tetrazol-5-yl)thiazol-2-amin (1) 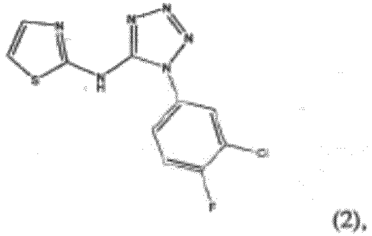 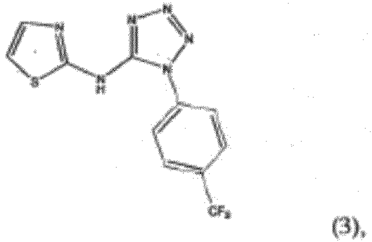 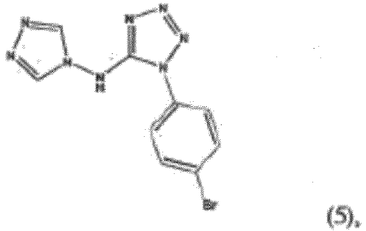 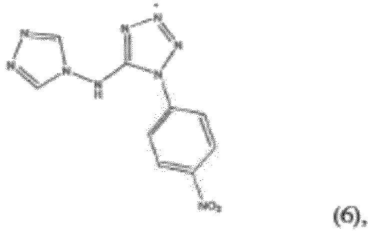 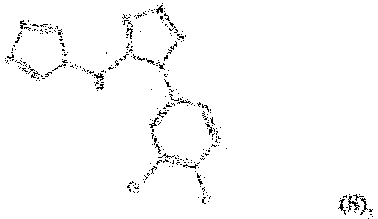 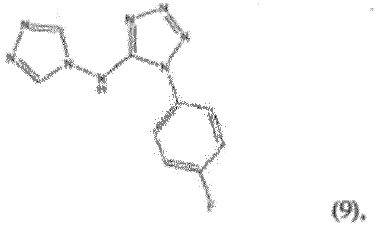 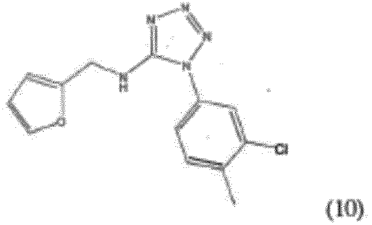
oder

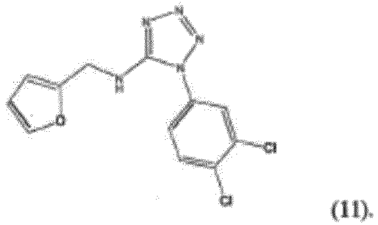

2. Verbindung gemäß Anspruch 1 zur Verwendung als Medikament.

3. Verbindung gemäß Anspruch 2 zur Verwendung als antibakterieller Wirkstoff.

4. Verbindung gemäß Anspruch 2 zur Verwendung als antimykotischer Wirkstoff.

## Revendications

1. Composé de formule I 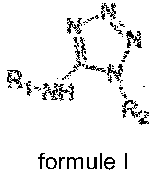 dans lequel R₁ et R₂ sont indépendamment un cycle aromatique, en particulier un cycle phényle ou hétéroaromatique, en particulier un triazole, thiadiazole ou furane, substitué par un halogène, spécialement un choisi parmi F, Cl, Br, ou un groupe méthyle, trifluorométhyle, méthoxy ou éthoxy,
dans lequel ledit composé a été choisi parmi :
la N-(1-(3,4-dichlorophényl)-1H-tétrazol-5-yl)thiazole-2-amine (1), 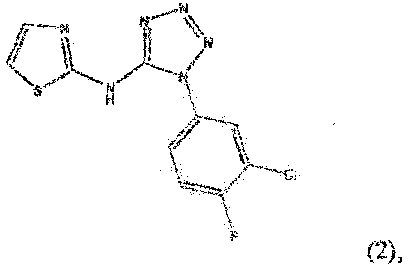 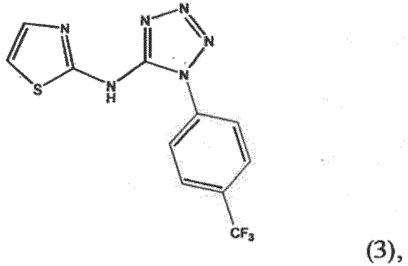 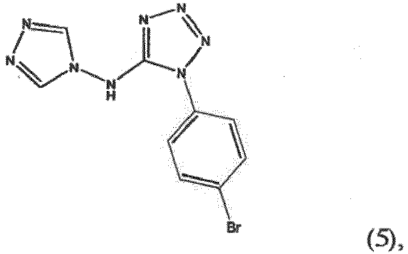 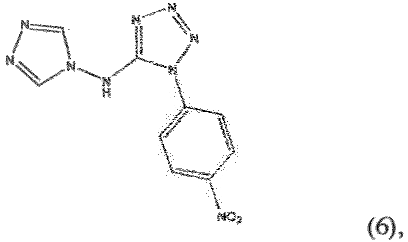 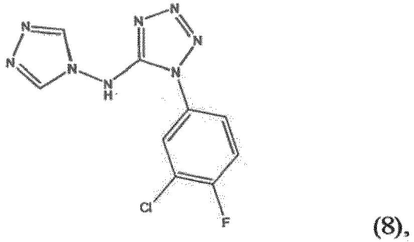 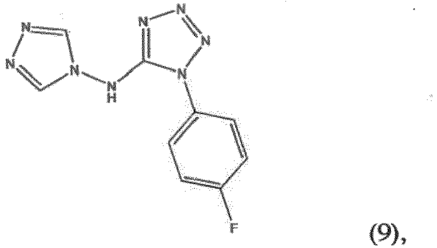 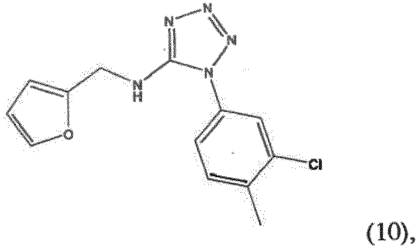
ou

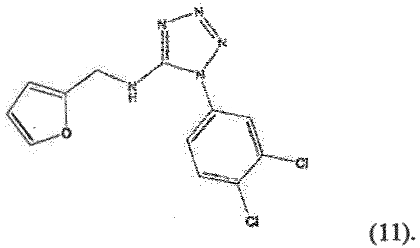

2. Composé selon la revendication 1, destiné à être utilisé comme médication.

3. Composé selon la revendication 2, destiné à être utilisé comme médicament antibactérien.

4. Composé selon la revendication 2, destiné à être utilisé comme médicament antifongique.
